# EUROPÄISCHE PATENTANMELDUNG

(11) **EP 2 338 534 A2**
(43) Veröffentlichungstag der Anmeldung: **29.06.2011**
(21) Anmeldenummer: 10192872.9
(22) Anmeldetag: 29.11.2010
(51) Int. Cl.: A61L 27/54, A61L 31/16

(54) **Medizinisches Implantat, Beschichtungsverfahren sowie Implantationsverfahren**

(30) Priorität: 21.12.2009 US 288346 P
(71) Anmelder: Biotronik VI Patent AG, 6341 Baar (CH)
(72) Erfinder: Harder, Claus, 91080, Uttenreuth (DE)
(74) Vertreter: Lindner-Vogt, Karin L.

(57) **Zusammenfassung**

Die Erfindung betrifft ein medizinisches Implantat, mit einem Grundkörper (12a, 12b), der ein erstes Ende (14a, 14b) und ein zweites Ende (16a, 16b) aufweist, die in einer Haupterstreckungsrichtung (18a, 18b) des Grundkörpers (12a, 12b) an sich gegenüberliegenden Enden des Grundkörpers (12a, 12b) angeordnet sind, und einer Beschichtung (20a, 20b), wobei die Beschichtung (20a, 20b) zumindest einen Wirkstoff (22a, 22b) mit einem Wirkstoffgradienten (24a, 24b) aufweist.

Es wird vorgeschlagen, dass eine Wirkstoffmenge (26a, 26b) zumindest in der Haupterstreckungsrichtung (18a, 18b) des Grundkörpers (12a, 12b) von dem ersten Ende (14a, 14b) zu dem zweiten Ende (16a, 16b) abnimmt.

## Beschreibung

Die Erfindung betrifft ein medizinisches Implantat sowie ein Verfahren, insbesondere ein Beschichtungsverfahren, zur Beschichtung eines medizinischen Implantats und ein Verfahren, insbesondere ein Implantationsverfahren, zur Implantation eines medizinischen Implantats in einen tierischen und/oder menschlichen Körper nach den Oberbegriffen der unabhängigen Ansprüche.

In der Medizin kommen Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina-Implantate, zahnmedizinische Implantate, Implantate zum Gelenkersatz, Gefäßprothesen oder Stents. Insbesondere wirkstoffbeschichtete Stents, so genannte Drug-Eluting Stents (DES) werden in der jüngsten Zeit im Bereich der Herz-Kreislauf-Erkrankungen immer häufiger eingesetzt, um im Anschluss an eine Aufweitung stenosierter Koronargefäße mittels einer Angioplastie und einer Stabilisierung durch eine Stentimplantation die Spätfolgen des Eingriffs, wie eine Wiederverengung oder einen Wiederverschluss der Gefäße, durch eine lokale Wirkstoffadministration zu reduzieren.

Bekannt ist aus der DE 10 1006 038239 A1, einen Stent vorzusehen, der eine lagenweise, so genannte Layer-by-Layer Beschichtung trägt, die einen radialen Wirkstoffgradienten aufweist, so dass ein Wirkstoff oder mehrere Wirkstoffe in unterschiedlicher und zeitabhängiger Dosierung bzw. in einem oder mehreren unterschiedlichen Zeitfenstern eluiert werden. Ferner offenbart dieser Stand der Technik ein Verfahren zur Herstellung des beschichteten Stents, bei dem der Wirkstoffgradient durch Auftragung von unterschiedlichen Wirkstoff/Polymer-Mischungen Schicht für Schicht in radialer Richtung erreicht wird. Auch aus der US 2005 0075714 A1 sind ein derartiger Stent sowie ein vergleichbares Verfahren bekannt.

Die US 2008 0195079 A1 offenbart einen Stent mit drei unterschiedlich beschichteten Bereichen, wobei die Bereiche an Enden des Stents dieselbe Wirkstoffkonzentration aufweisen, die höher ist als eine Wirkstoffkonzentration in einem Bereich zwischen den beiden Enden.

Der Erfindung liegt die Aufgabe zugrunde, ein medizinisches Implantat zu schaffen sowie ein Verfahren zur Implantation eines medizinischen Implantats in einen tierischen und/oder menschlichen Körper bereit zu stellen, das jeweils eine gute Wirkstoffapplikation und eine gezielte Wirkungsweise ermöglicht. Ferner hat die Erfindung die Aufgabe, ein Verfahren zur Beschichtung eines medizinischen Implantats bereit zu stellen, das eine Beschichtung konstruktiv einfach ermöglicht.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Zeichnung und der Beschreibung.

Die Erfindung geht aus von einem medizinischen Implantat, mit einem Grundkörper, der ein erstes Ende und ein zweites Ende aufweist, die in einer Haupterstreckungsrichtung des Grundkörpers an sich gegenüberliegenden Enden des Grundkörpers angeordnet sind, und einer Beschichtung, wobei die Beschichtung zumindest einen Wirkstoff mit einem Wirkstoffgradienten aufweist.

Wesentlich ist hierbei die Erkenntnis, dass die an einer bestimmten Stelle der Gefäßwand deponierte Wirkstoffmenge nicht nur von der lokalen Elution des Wirkstoffs an eben dieser Stelle abhängt, sondern zusätzlich durch das den Stent durchströmende Medium beeinflusst wird, da dieses Medium proximal von dieser Stelle in den Strom gelangenden Wirkstoff stromabwärts transportiert und an dieser Stelle ablagern kann. Insbesondere bei der Verwendung von Wirkstoffen mit schmalem therapeutischem Fenster spielt dieser Effekt eine Rolle, die bei der Erreichung einer gezielten Wirkstoffdeposition berücksichtigt werden muss.

Es wird vorgeschlagen, dass eine Wirkstoffmenge zumindest in der Haupterstreckungsrichtung des Grundkörpers von dem ersten Ende zu dem zweiten Ende abnimmt. In diesem Zusammenhang soll unter dem Begriff "abnehmen" insbesondere eine kontinuierlich oder quasikontinuierlich Abnahme an Wirkstoffmenge verstanden werden, die stufenweise, monoton und/oder streng monoton fällt. Unter einem "Ende des Grundkörpers" soll hier insbesondere ein Bereich des Implantats verstanden werden, an den sich nur in einer Richtung eine Implantatstruktur bzw. Grundkörperstruktur, wie beispielsweise ein Drahtgeflecht, anschließt. Hier soll unter einer "Haupterstreckung" insbesondere eine Länge des Grundkörpers und/oder in einem implantierten Zustand des Implantats eine Richtung entlang einer Fließrichtung eines Fließmediums verstanden werden. Unter "sich gegenüberliegenden Enden" sollen hier insbesondere Enden verstanden werden, die relativ zur Fließrichtung an unterschiedlichen Enden bzw. am Anfang und am Ende des Implantats angeordnet sind. Im implantierten Zustand des Implantats in einem von einem Fließmedium durchflossenen Hohlkörper eines tierischen und/oder menschlichen Körpers, kann somit die Wirkstoffinenge in der Beschichtung in axialen Fließrichtung je nach Implantationsrichtung entweder ab oder zu nehmen. Durch die erfindungsgemäße Ausgestaltung wird ein medizinisches Implantat bereitgestellt, das abgestimmt ist auf die Parameter der Implantationsstelle, wie eine Wirkstoffabsorption einer an dem Implantat angrenzenden und/oder anliegenden Hohlraumwand und/oder einer Fließgeschwindigkeit eines durch den Hohlraum bzw. das Implantat durchfließenden Fließmedium. Dadurch stellt sich im implantierten Zustand im Implantationsbereich eine gewünschte und bevorzugt homogene Wirkstoffkonzentration in der umliegenden Hohlraumwand ein, wodurch besonders vorteilhaft lokaler Über- bzw. Unterdosierungen vermieden werden.

Ferner soll unter einem "Implantat" insbesondere ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinenden medizinischen Implantate, wie beispielsweise ein Herzschrittmacher, ein Hirnschrittmacher, ein Herzimplantat, ein Cochleaimplantat, ein Retina-Implantat, ein zahnmedizinisches Implantat, ein Implantat zum Gelenkersatz, eine Gefäßprothesen oder besonders vorteilhaft wird eine Ausbildung des medizinischen Implantats als ein Stent, insbesondere ein Koronarstent, vorgeschlagen. Das Implantat bzw. der Stent umfasst bevorzugt ein Depot für einen Arzneiwirkstoff, wodurch eine Dosierung des Wirkstoffs vorteilhaft auf die Bedürfnisse einer den Stent umschließenden Gefäßwand mit Berücksichtigung einer Blutfliesgeschwindigkeit abgestimmt werden kann.

Ferner soll in diesem Zusammenhang unter einem "Grundkörper" insbesondere eine Struktur, wie beispielsweise ein Drahtgeflecht, verstanden werden, die im Wesentlichen eine Gestalt und/oder eine Form des Implantats bzw. des Stents bildet. Zudem ist der Grundkörper vorzugsweise aus einem metallischen Material und/oder aus einer Kombination von mehreren metallischen Materialien gefertigt, wie beispielsweise Eisen, Magnesium, Nickel, Wolfram, Titan, Zirkonium, Niob, Tantal, Zink, Silizium, Lithium, Natrium, Kalium, Kalzium, Mangan und/oder jedem anderen, dem Fachmann als sinnvoll erscheinenden Material. Möglich wären auch eine Zink-Kalziumlegierung und/oder ein Formgedächtnis-Material, wie beispielsweise eine Nickel-Titan-Legierung und/oder Kupfer-Zink-Aluminium-Legierung, vorzugsweise Nitinol. Des Weiteren kann es vorteilhaft sein, wenn der Grundkörper zumindest Kobalt und/oder Chrom aufweist, vorzugsweise in der Form von Edelstahl und/oder eines Cr-Ni-Fe-Stahl - hier bevorzugt die Legierung 316L - oder einem Co-Cr-Stahl. Durch diese Ausgestaltung kann ein Implantat bereitgestellt werden, das zu zufrieden stellenden Beschichtungsergebnissen führt sowie eine gute Dilatierbarkeit und eine vorteilhafte Flexibilität gepaart mit einer hohen Stabilität aufweist.

Grundsätzlich wäre es jedoch auch denkbar, dass der Grundkörper des Implantats mindestens teilweise aus Kunststoff, einer Keramik und/oder aus einem biodegradierbaren Material besteht.

Zudem soll in diesem Zusammenhang unter einer "Beschichtung" insbesondere eine zumindest teilweise Ummantelung des Implantats, vorzugsweise eines Stents, und/oder eine Befüllung und/oder eine Beladung von zumindest einer Aussparung, vorzugsweise in der Form eines Lochs, einer Kavität und/oder eines Körbchens im und/oder am Implantat, vorzugsweise Stent, mit einer geeigneten Matrix, die zumindest einen Wirkstoff umfasst, verstanden werden. Bevorzugte Polymere für eine Polymermatrix des erfindungsgemäßen Implantats werden ausgewählt aus der Gruppe:
- nichtresorbierbarer/permanenter Polymere wie beispielsweise:
   Polypropylen, Polyethylen, Polyvinylchlorid, Polyacrylate (Polyethyl- und Polymethylacrylate, Polymethylmetacrylat, Polymethylcoethylacrylat, Ethylen/Ethylacrylat) Polytetrafluorethylen (Ethylen/Chlortrifluorethylen Copolymer, Ethylen/Tetrafluorethylen Copolymer), Polyamide (Polyamidimid, PA-11, -12, -46, -66), Polyetherimid, Polyethersulfon, Poly(iso)butylen, Polyvinylchlorid, Polyvinylfluorid, Polyvinylalkohol, Polyurethan, Polybuthylenterephthalat, Silikone, Polyphosphazen, Polymerschäume (aus z.B. Carbonaten, Styrolen), sowie die Copolymere und Elends der aufgezählten Klassen, bzw. der Klasse der Thermoplaste und der Elastomere im Allgemeinen.
- resorbierbarer/bioresorbierbarer/degradierbarer Polymere wie beispielsweise: Polydioxanon, Polyglycolid, Polycaprolacton, Polylactide [Poly-L-Lactid, Poly-D,L-Lactid, und Copolymere sowie Elends wie Poly(L-Lactid-co-glycolid), Poly(D,L-lactid-co-glycolid), Poly(L-Lactid-co-D,L-Lactid), Poly(I-Lactid-co-trimethylencarbonat)], Triblockcopolymere, Polysaccharide [Chitosan, Levan, Hyaluronsäure, Heparin, Dextran, Cellulose etc.], Polyhydroxyvalerat, Ethylvinylacetat, Polyethylenoxid, Polyphosphorylcholin, Fibrin, Albumin, Polyhydroxybuttersäure (ataktisch, isotaktisch, syndiotaktisch sowie deren Elends) etc.

Besonders bevorzugt enthält die Beschichtung zumindest ein Polylactid, wie insbesondere Poly-L-Lactid, wodurch eine biokompatible und besonders gewichtsarme Beschichtung bereitgestellt werden kann.

Unter einem "Wirkstoff" soll hier insbesondere eine Substanz verstanden werden, die im tierischen und/oder menschlichen Körper eine biochemische bzw. biologische Reaktion hervorruft und/oder unterbindet. In diesem Sinne kann Wirkstoff auch synonym zu Arzneistoff und/oder Pharmakon verwendet werden. Es ist vorteilhaft, wenn der zumindest eine Wirkstoff ausgewählt ist aus einer Gruppe bestehend aus: Lipidregulatoren (Fibrate), Immunsuppressiva, Immunmodulatoren, Vasodilatatoren (Sartane), Calciumkanalblocker, Calcineurininhibitoren (Tacrolimus), Antiphlogistika (Glucocorticoiden, Cortison, Dichlofenac), Antünflammatorika (Imidazole), Antiallergika, Oligonucleoptide (dODN), Östrogene (Genistein), Endothelbildner (Fibrin), Steroide, Proteine/Peptide, Proliferationshemmer, Analgetika, Antirheumatika, Zytostatika. Dadurch kann ein medizinisches Implantat bzw. ein Stent mit einem breiten Anwendungsspektrum zur Verfügung gestellte werden.

In einer weiteren Ausgestaltung erweist sich die Erfindung insbesondere bei der Verwendung eines Wirkstoffs mit einem schmalen therapeutischen Fenster als vorteilhaft. Hierbei soll unter der Wendung "schmales therapeutisches Fenster" insbesondere eine schmale therapeutische Breite verstanden werden, wobei die "therapeutische Breite" ein Verhältnis zwischen einer therapeutischen Dosis und einer toxischen Dosis des Wirkstoffs ist. Durch das erfindungsgemäße Beschichtungsprofil des medizinischen Implantats können Gefahren von Substanzen mit einem schmalen therapeutischen Fenster, die beispielsweise kritischen Grenzen bzgl. einer Über- oder Unterdosierung aufweisen, vorteilhaft minimiert werden. Eine besonders gute Wirkungsweise und eine häufige Anwendung kann vorteilhaft erreicht werden, wenn es sich bei dem zumindest einen Wirkstoff um das Immunsuppressivum Sirolimus handelt.

Des Weiteren wird vorgeschlagen, dass der Wirkstoffgradient durch eine Dicke der Beschichtung eingestellt ist. Hierbei definiert "Dicke" eine radiale Erstreckung der Beschichtung bezogen auf einen Mittelpunkt des Implantats bzw. einen Mittelpunkt einer Kreisfläche des Stents. Unter einem "Wirkstoffgradient" soll hier insbesondere eine Änderung der Wirkstoffmenge zwischen zwei Punkten und insbesondere zwischen dem ersten Ende und dem zweiten Ende verstanden werden. Bevorzugt sind eine Dicke der Beschichtung und damit auch eine Wirkstoffmenge an dem ersten Ende größer in Bezug auf eine Dicke bzw. Wirkstoffmenge an dem zweiten Ende des Grundkörpers. Durch die variable, bevorzugt monoton schmäler werdende Dicke entlang der Länge in der Haupterstreckungsrichtung des Grundkörpers bzw. Implantats kann der Wirkstoffgradient konstruktiv einfach eingestellt und/oder hergestellt werden.

Vorteilhafterweise ist der Wirkstoffgradient mittels einer oder mehrerer Wirkstoffkonzentrationen entlang der Beschichtung eingestellt. Hierbei ist die radiale Beschichtungsdicke entlang der Haupterstreckung des Implantats über die gesamte Länge gleich, jedoch die Wirkstoffmengen unterscheiden sich an dem ersten Ende und dem zweiten Ende. Durch die Realisierung von mehreren Wirkstoffkonzentrationen kann das Implantat vorteilhaft in seinen Abmessungen homogen ausgestaltet und/oder hergestellt werden.

Ferner wird vorgeschlagen, dass das erste Ende in einem implantierten Zustand in einem Hohlraum als stromaufwärtiges Ende in einer axialen Fließrichtung anordenbar ist. In diesem Zusammenhang soll unter einem "Hohlraum" insbesondere ein Hohlraum in einem tierischen und/oder menschlichen Körper, wie eine röhrenförmige Struktur und/oder ein tubuläres Gefäß, verstanden werden, durch das ein fließfähiges Medium und/oder eine Körperflüssigkeit, wie beispielsweise Lymphe, Galle, Urin, Tränenflüssigkeit, Speichel, Liquor und/oder insbesondere Blut, fließt. Unter einem "stromaufwärtigen Ende" soll hier insbesondere ein proximales Ende und/oder ein Ende verstanden werden, das zu einem früheren Zeitpunkt mit dem Fließmedium in Kontakt kommt als ein weiteres und/oder das zweite Ende und/oder ein distales Ende. Im implantierten Zustand liegt das Implantat bevorzugt an einer Innenfläche einer Wand des Hohlraums und/oder einer Gefäßwand zumindest teilweise zu einer Abstützung dieser an. Durch diese erfindungsgemäße Orientierung des Implantats und somit des Beschichtungsprofils ist dieses besonders strömungsoptimiert ausgestaltet.

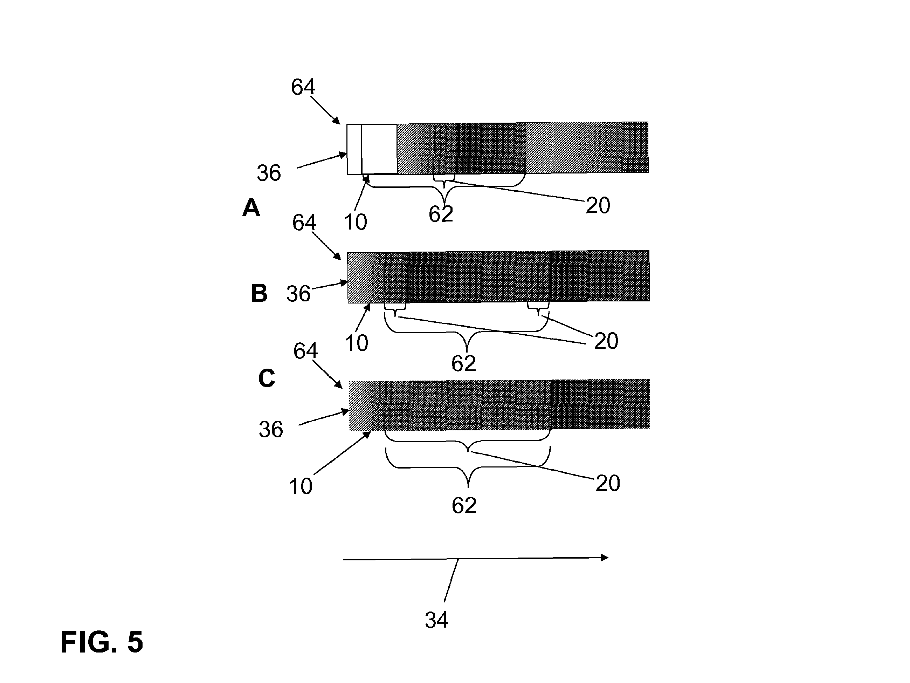

Weist das medizinische Implantat eine Ausgestaltung zur Eluierung zumindest eines Wirkstoff in einem menschlichen und/oder tierischen Körper auf, wobei der zumindest eine Wirkstoff derart eluierbar ist, dass sich eine gewünschte lokale Konzentrationsverteilung in einer angrenzenden Hohlraumwand ergibt, kann ein besonders therapeutisch wertvolles Implantat mit einem besonders ausgewogenen Wirkungsspektrum bzw. ―Potenzial bereitgestellt werden. Hierbei soll unter der Wendung "gewünschte lokale Konzentrationsverteilung" insbesondere verstanden werden, dass eine Konzentrations- bzw. Wirkstoffverteilung auf zumindest einen Parameter des Implantat/Hohlraumsystems abgestimmt ist. Der Parameter kann sich hierbei auf den Wirkstoff, auf den Hohlraum, auf die Hohlraumwand und/oder auf das den Hohlraum umgebenden und/oder durchfließenden Medium bzw. Fließmedium beziehen und eine Wirkstoffart, ein Volumen, eine Resorbierfähigkeit, eine Elastizität, eine Fließgeschwindigkeit etc. darstellen. Die Wirkstoffverteilung kann inhomogen oder vorteilhafterweise homogen sein. Die gewünschte lokale Konzentrationsverteilung einer jeweiligen Wirkstoffverteilung in der Beschichtung kann durch Simulation ermittelt und/oder optimiert werden (siehe auch Figur 5).

Verschiedene Arten der Elution des Wirkstoffs bzw. einer Wirkstofffreisetzung aus der Beschichtung bzw. deren Polymermatrix werden entsprechend dem Design des erfindungsgemäßen Implantates aus Implantatgrundkörper, Trägermatrix und Wirkstoff unterschieden:
- Wirkstoffe können unabhängig voneinander kovalent oder aufgrund ionischer oder van der Waals Wechselwirkung direkt auf dem Implantatgrundkörper aufgebracht werden, zum Beispiel auf einen metallischen Stentgrundkörper.
- Denkbar ist auch die kovalente Anbindung des oder der Wirkstoffe auf einer Beschichtung. Eine Freisetzung erfolgt dann über hydrolytische oder enzymatische Spaltung der Bindung.
- Wirkstoffe können in Kavitäten im Stentmaterial eingebracht und mit einer polymeren Deckschicht versehen werden. Die Elution erfolgt entweder über Diffusionsvorgänge durch die Deckschicht oder im Falle einer sich nach Implantation sofort auflösenden

Schicht, direkt.
- Der Wirkstoff befindet sich in einer porösen, meist anorganischen Trägermatrix und wird von dieser ins Gewebe abgegeben.
- Eine Wirkstofffreisetzung kann über Quellung seiner Trägermatrix erfolgen.
- Durch Erosion der Trägermatrix biodegradierbarer Polymere kann eine Eluierung erzielt werden.
- Der Wirkstoff, der zwischen zwei polymeren Schichten eingebettet ist, eluiert aus dieser Sandwichstruktur über diffusive Vorgänge.
- Ein Elend aus Wirkstoff und Polymer wird auf dem Implantatgrundkörper, insbesondere Stentgrundkörper, aufgebracht und somit wird die Freisetzung über Diffusion ermöglicht.
- Die Verwendung eines Topcoats, d.h. einer zusätzlichen Polymerschicht ohne Wirkstoff in Kooperation, über einer wirkstoffinkorporierten Polymerschicht, führt zur Retadierung des Elutionsvorganges.

Ferner geht die Erfindung aus von einem Verfahren, insbesondere einem Beschichtungsverfahren, zur Beschichtung eines medizinischen Implantats mit einem Grundkörper, der ein erstes Ende und ein zweites Ende aufweist, und einer Beschichtung, wobei die Beschichtung zumindest einen Wirkstoff mit einem Wirkstoffgradienten aufweist.

Es wird vorgeschlagen, dass ein Beschichtungsstrahl mit einem Polymer/Wirkstoffgemisch mit einer variablen Geschwindigkeit von dem ersten Ende zu dem zweiten Ende bewegt wird. In diesem Zusammenhang soll unter einem "Polymer/Wirkstoffgemisch" jede, dem Fachmann als sinnvoll erscheinende Kombination von einem und/oder mehreren bereits aufgeführten Polymeren und/oder einem und/oder mehreren bereits aufgeführten Wirkstoffen verstanden werden. Unter einer "variablen Geschwindigkeit" soll hier insbesondere eine lineare oder verändert lineare Geschwindigkeit verstanden werden. Ein Implantat ist beispielsweise ein Stent und bevorzugt ein Koronarstent. Durch die sich verändernde Geschwindigkeit werden entlang einer Haupterstreckung vom ersten Ende zum zweiten Ende bzw. einer Länge des Implantats unterschiedliche Beschichtungsdicken aufgetragen, wodurch sich an unterschiedlichen axialen Positionen des Implantats bzw. Stents unterschiedliche Wirkstoffmengen einstellen. Der Beschichtungsstrahl kann von jeder, dem Fachmann denkbaren Beschichtungseinheit, beispielsweise ausgestatte mit einer Zwei-Stoff-Düse (mit oder ohne Zwangsförderung), einer Ein-Stoff-Düse, einer Rückpralldüse, einem Dosierventil, einem Rotationszerstäuber und/oder einem Ultraschallzerstäuber, generiert werden. Generell wäre auch eine Bewegung des Implantats relativ zu dem Beschichtungsstrahl denkbar. Ferner wäre auch jedes andere, dem Fachmann als zweckdienlich erscheinende Verfahren zur Beschichtung des Implantats, wie beispielsweise mittels eines Walzenauftrags und/oder mittels eines Tauchreservoirs, denkbar. Es wäre grundsätzlich auch möglich, eine Konzentration des Wirkstoffs im Polymer/Wirkstoffgemisch durch unterschiedliche Mischungsbedingungen zu variieren. Durch die erfindungsgemäße Ausgestaltung kann der Wirkstoffgradient konstruktiv einfach eingestellt und/oder hergestellt werden. Zudem kann die Beschichtung verlässlich und prozesssicher aufgebracht werden.

In einer alternativen Ausführung kann vorgesehen sein, dass der Beschichtungsstrahl mit zunehmender Geschwindigkeit von dem ersten Ende zu dem zweiten Ende bewegt wird. Dadurch wird die radiale Dicke der Beschichtung ausgehend von einer Beschichtungsdicke am ersten Ende, dem proximalen Ende im implantierten Zustand des beschichteten Implantats, hin zum zweiten Ende, dem distalen Ende, entlang der Länge dünner, wodurch auch die Wirkstoffmenge, bevorzugt stetig monoton fallend, vom ersten zum zweiten Ende weniger bzw. geringer wird. Mittels der Zunahme der Geschwindigkeit kann eine Beschichtung mit einem monoton fallenden Wirkstoffgradient mit einfachen Mitteln schnell und zuverlässig aufgetragen werden.

Eine bevorzugte Weiterbildung besteht darin, dass der Beschichtungsstrahl relativ zu dem medizinischen Implantat in axialer Richtung und/oder in Umfangsrichtung bewegt wird. Hierbei wird bevorzugt der Beschichtungsstrahl nach einem Umlaufen des Implantats um den gesamten Umfang des Implantats bzw. um 360° um das Implantat um eine Länge Δz axial bzw. ausgehende vom ersten Ende in Richtung des zweiten Endes bewegt. Hierbei ist Δz lediglich limitiert durch die konstruktiven Vorgaben der Beschichtungsvorrichtung. Durch die Realisierung der Bewegung in axialer und/oder in Umfangsrichtung kann die Beschichtung des Implantats vorteilhaft homogen und gleichmäßig gestaltet werden.

Zudem geht die Erfindung aus von einem Verfahren, insbesondere einem Implantationsverfahren, zur Implantation eines medizinischen Implantats in einem tierischen und/oder menschlichen Körper mit einem ersten Ende und einem zweiten Ende, wobei das erste Ende eine höhere Wirkstoffbeladung aufweist als das zweite Ende.

Es wird vorgeschlagen, dass das erste Ende in einem Hohlraum als stromaufwärtiges Ende in einer axialen Fließrichtung angeordnet wird. Unter einer "Wirkstoffbeladung" soll hier insbesondere eine Wirkstoffmenge pro Fläche verstanden werden. Das Implantat stellt eine Stent und bevorzugt einen Koronarstent dar. Der Hohlraum wird von einem Gefäß, insbesondere von einem Blutgefäß und besonders bevorzugt von einer Koronararterie gebildet. Als Wirkstoff kommt jede, dem Fachmann als geeignet erscheinende Substanz und insbesondere eine und/oder mehrere der eingangs genannten Substanzen in betracht. Durch die erfindungsgemäße Ausgestaltung kann eine besonders ausgereifte Therapieform zur Verfügung gestellt werden, die insbesondere auf die *in vivo* Bedürfnisse einer Implantationsstelle abgestimmt ist.

Die Erfindung ist nachfolgend beispielhaft, anhand von in Zeichnungen dargestellten Ausführungsbeispielen, näher erläutert. Es zeigen in schematischer Darstellung:
Fig. 1 eine erfindungsgemäßes medizinisches Implantat in einer Aufsicht mit einer Beschichtung,
Fig. 2 eine schematische Darstellung eines Schnitts durch das medizinische Implantat der Figur 1 mit einer Beschichtung in einer ersten Ausführungsform,
Fig. 3 eine Diagrammdarstellung eines Wirkstoffgradienten,
Fig. 4 das Implantat der Figur 1 implantiert in einem Hohlraum,
Fig. 5 a-c drei Stimulationen einer Elution eines Wirkstoffs dreier unterschiedlich beschichteter medizinischer Implantate nach dem Stand der Technik,
Fig. 6 eine schematische Darstellung eines Beschichtungsverfahrens des medizinischen Implantats der Figur 1 und
Fig. 7 eine schematische Darstellung eines Schnitts durch ein medizinische Implantat mit einer alternativen Ausführungsform der Beschichtung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verweisen.

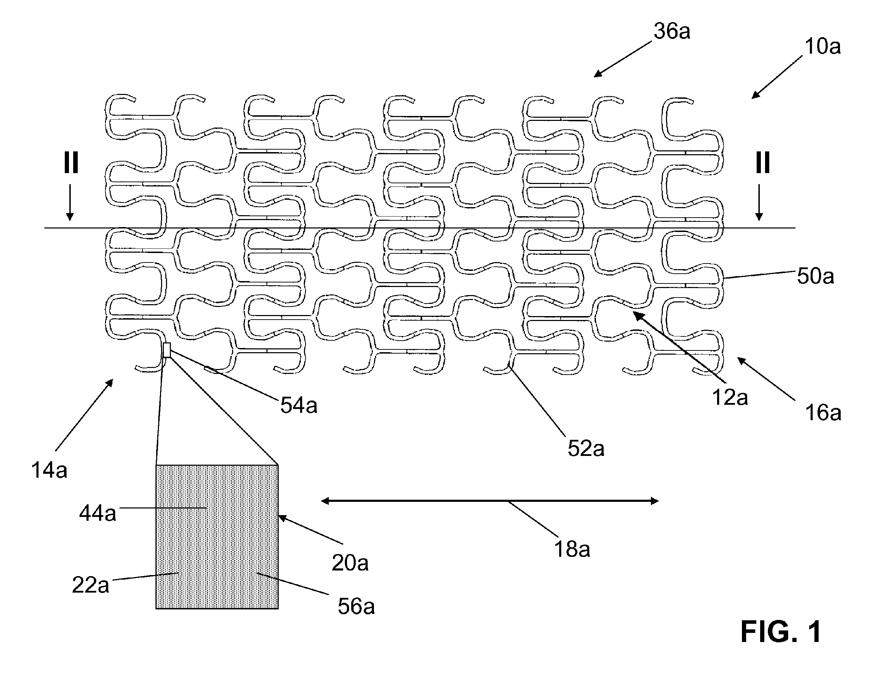

Fig. 1 zeigt in einer Aufsicht ein medizinisches Implantat 10a, in der Form eines Stents 36a bzw. Koronarstents, mit einem aus Chrom-Kobalt-Stahl gefertigten Grundkörper 12a, der als Grundkörperstruktur ein Drahtgeflecht 50a, ausgebildet von Stentstruts 52a, aufweist. Ferner weist der Grundkörper 12a ein erstes Ende 14a und ein zweites Ende 16a auf, die in einer Haupterstreckungsrichtung 18a des Grundkörpers 12a an sich gegenüberliegenden Enden des Grundkörpers 12a angeordnet sind. Generell kann die Ausgestaltung des Drahtgeflechts 50a jede beliebige, dem Fachmann als sinnvoll erscheinende Form sein.

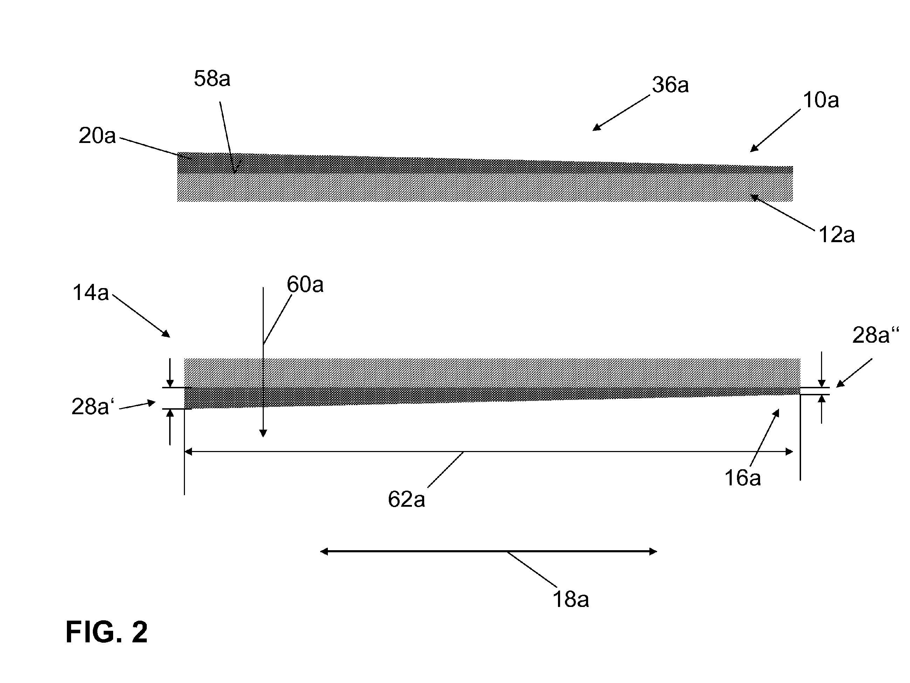

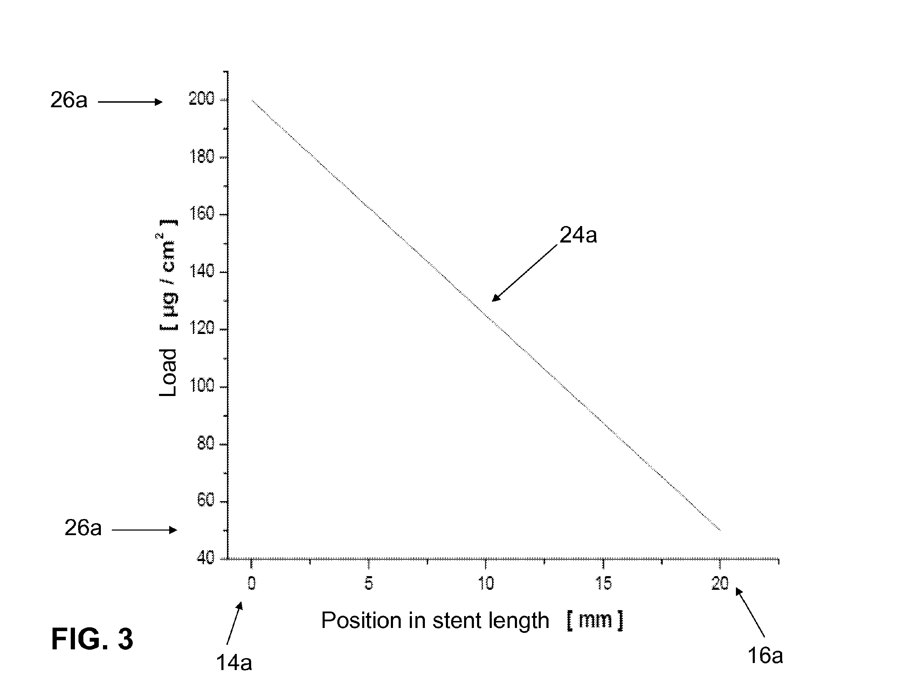

Das Implantat 10a bzw. der Stent 36a ist zudem mit einer Beschichtung 20a beschichtet, die in Kavitäten 54a der Stentstruts 52a eingebracht ist. Diese Kavitäten 54a sind in dem Fachmann bekannter Weise über die gesamte Fläche der Stentstruts 52a verteilt angeordnet, hier ist jedoch zur übersichtlicheren Darstellung nur eine Kavität 54a angedeutet und symbolisch vergrößert gezeigt. Die Beschichtung 20a ist von einem Polymer/Wirkstoffgemisch 44a gebildet, das als Wirkstoff 22a das Immunsuppressivum Sirolimus und als Polymermatrix 56a Poly-L-Lactid (PLLA) aufweist. Somit hat der Wirkstoff 22a ein schmales therapeutisches Fenster. Ferner weist der Wirkstoff 22a der Beschichtung 20a einen Wirkstoffgradienten 24a auf (Figuren 2 und 3).

In der Fig. 2 ist das medizinische Implantat 10a schematisch in einer Schnittdarstellung entlang der Linie II-II in Figur 1 gezeigt. Hierbei ist die Beschichtung 20a zur Verdeutlichung des Beschichtungsprofils auf einer Außenfläche 58a des Grundkörpers 12a symbolisch dargestellt. Selbstverständlich können auch die Innen- und Seitenflächen der Stentstruts beschichtet sein, was in der Zeichnung der Übersichtlichkeit halber aber nicht dargestellt ist. Die Beschichtung 20a weist in einer radialen Richtung 60a unterschiedliche Dicken 28a', 28a" entlang der Haupterstreckungsrichtung 18a auf, wodurch ein Wirkstoffgradient 24a durch die Dicke 28a', 28a" der Beschichtung 20a eingestellt ist. Am ersten Ende 14a des Grundkörpers 12a ist die Dicke 28a' der Beschichtung 20a dicker als die Dicke 28a" am zweiten Ende 16a. Da die Polymer/Wirkstoff-Matrix der Beschichtung 20a über eine gesamte Länge 62a des Grundkörpers 12a die gleiche Wirkstoffkonzentration aufweist, nimmt eine Wirkstoffmenge 26a in der Haupterstreckungsrichtung 18a des Grundkörpers 12a von dem ersten Ende 14a zu dem zweiten Ende 16a ab bzw. fällt streng monoton (Figur 3).

In der Fig. 3 ist eine Diagrammdarstellung des Wirkstoffgradienten 24a gezeigt. Hierbei ist auf der x-Achse die Position der Stentlänge in mm und auf der y-Achse die Beladung in µg Wirkstoff pro cm² Stentfläche aufgetragen. Es ist deutlich zu sehen, dass die Beschichtung an einem Anfang des Implantats 10a bzw. dem ersten Ende 14a bei Position 1 mm eine Beschichtung von ca. 200 µg/cm² und an einem Ende des Implantats 10a bzw. am zweiten Ende 16a bei 20 mm eine Beschichtung von ca. 50 µg/ cm² aufweist. Die Wirkstoffmengen 26a nehmen ab und damit auch der Wirkstoffgradient 24a von dem ersten Ende 14a zu dem zweiten Ende 16a und zwar streng monoton fallend.

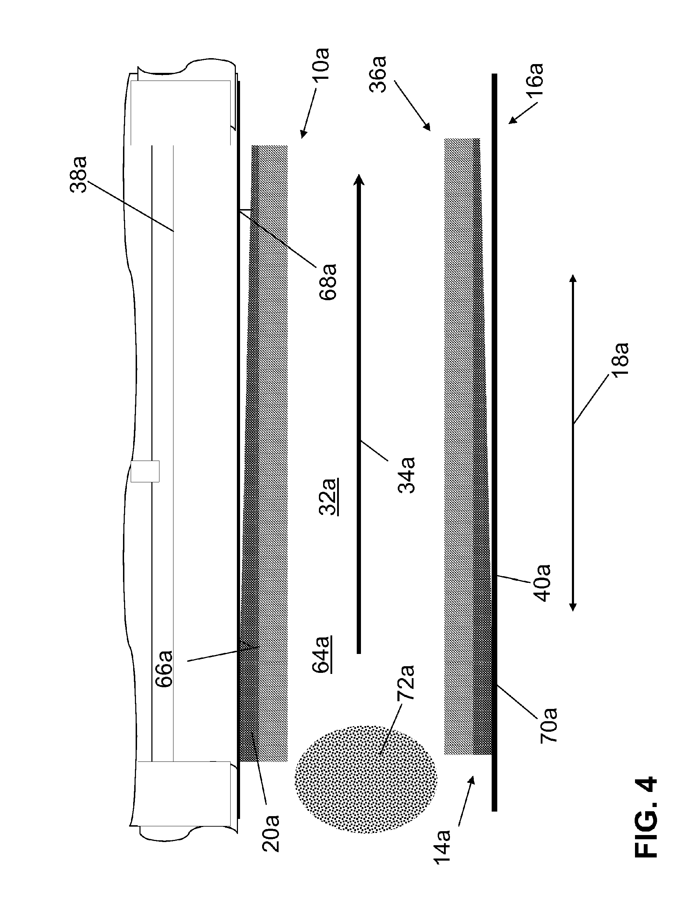

In Fig. 4 ist das medizinische Implantat 10a bzw. der Stent 36a in einem implantierten Zustand in einem Hohlraum 32a eines menschlichen Körpers 38a gezeigt. Das Implantat 10a wird mittels eines Verfahrens zur Implantation des medizinischen Implantats 10a bzw. einem Implantationsverfahren in den menschlichen Körper 38a eingebracht. Der Hohlraum 32a wird von einem tubulären Gefäß bzw. von einer Koronararterie 64a gebildet. Das Implantat 10a liegt dabei mit seiner Außenmantelfläche 66a an einer Innenfläche 68a einer Hohlraumwand 40a bzw. Gefäßwand 70a der Koronararterie 64a an (hier durch die symbolische und überdimensionale Darstellung der Beschichtung 20a nicht richtig dargestellt). Das erste Ende 14a, das eine höhere Wirkstoffbeladung aufweist als das zweite Ende 16a, wird in den Hohlraum 32a bzw. die Koronararterie 64a so eingebracht, dass es als stromaufwärtiges Ende in einer axialen Fließrichtung 34a eines Fließmediums 72a, das den Hohlraum 32a durchfließt und von einer Körperflüssigkeit bzw. von Blut gebildet wird, angeordnet ist.

Das medizinisches Implantat 10a weist eine Ausgestaltung zur Eluierung des Wirkstoffs 22a in dem menschlichen Körper 38a auf, wobei der Wirkstoff 22a derart eluierbar ist, dass sich eine gewünschte lokale Konzentrationsverteilung in der angrenzenden Hohlraumwand 40a ergibt. Diese gewünschte lokale Konzentrationsverteilung der jeweiligen Wirkstoffverteilung in der Beschichtung 20a kann durch Simulation ermittelt und/oder optimiert werden.

Fig. 5 zeigt exemplarisch drei Stimulationen einer Elution eines Wirkstoffs dreier unterschiedlich beschichteter medizinischer Implantate nach dem Stand der Technik (http://medtechinsider.com/?p=4000). Eine solche Stimulation wäre auch für den nach der Erfindung beschichteten Stent 36a denkbar. Die Elution wird exemplarisch für Beispiel C beschrieben. Ein medizinisches Implantat 10 bzw. ein Stent 36 ist in einer Koronararterie 64 implantiert und wird von einer Körperflüssigkeit (nicht gezeigt) in Fließrichtung 34 durchströmt. Ferner ist der Stent 36 über seine gesamte Länge 62 mit einer Beschichtung 20 versehen. Die in der Gefäßwand deponierte Wirkstoffmenge ist mittels einer Grauschattierung dargestellt. Zu sehen ist, dass in Fließrichtung 34 vor dem Stent 36 eine geringe Wirkstoffmenge durch Elution und Diffusion vorhanden ist. Im Bereich des Stents 36 steigt die Elution der Wirkstoffmenge über seiner gesamten Länge 62 an, die dann nach dem Stent 36 ausdünnt. Durch eine solche Simulation lässt sich die Beschichtung auf Parameter des Implantat/Hohlraumsystem abstimmen.

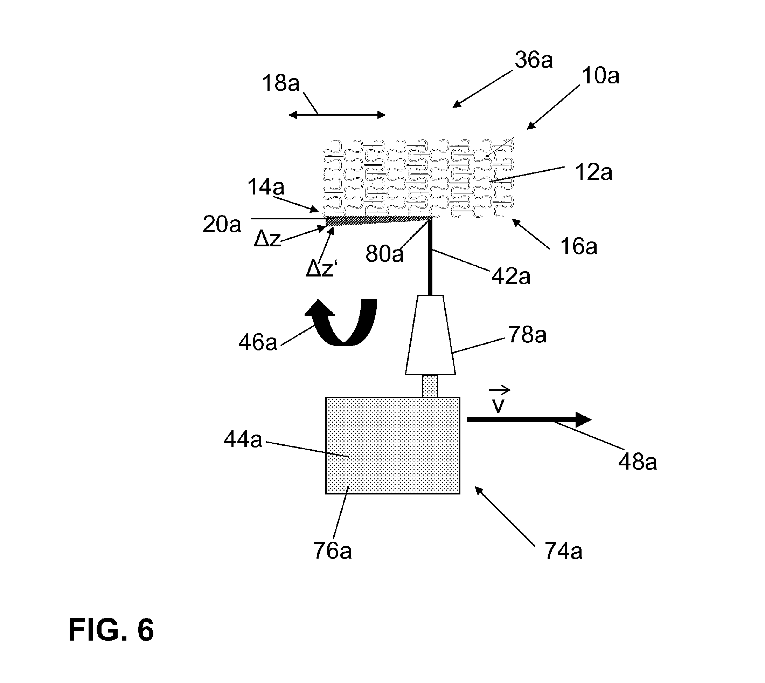

Die Fig. 6 zeigt schematisch eine Vorrichtung 74a für ein Verfahren zur Beschichtung bzw. ein Beschichtungsverfahren für ein medizinisches Implantat 10a, in der Form eines Stents 36a, mit einem Grundkörper 12a, der ein erstes Ende 14a und ein zweites Ende 16a aufweist. Ferner hat der Grundkörper 12a eine Beschichtung 20a, die einen Wirkstoff 22a mit einem Wirkstoffgradienten 24a aufweist. Die Vorrichtung 74a weist ein Reservoir 76a mit dem Polymer/Wirkstoffgemisch 44a auf, das mittels einer Düse 78a als ein Beschichtungsstrahl 42a auf das Implantat 10a bzw. den Stent 36a aufgebracht wird. Hierbei wird der Beschichtungsstrahl 42a aus dem Polymer/Wirkstoffgemisch 44a mit einer variablen bzw. mit einer zunehmenden Geschwindigkeit von dem ersten Ende 14a zu dem zweiten Ende 16a des Grundkörpers 12a bewegt. Durch die Geschwindigkeitszunahme wird die Beschichtung 20a von einer ersten Position bzw. dem ersten Ende 14a zu einer zweiten Position 80a bis hin zum zweiten Ende 16a immer dünner (Beschichtungsergebnis am zweiten Ende 16a nicht gezeigt). Um eine vollständige Beschichtung des Implantats 10a zu erreichen wird der Beschichtungsstrahl 42a mittels der Vorrichtung 74a relativ zu dem medizinischen Implantat 10a in Umfangsrichtung 46a und in axialer Richtung 48a bewegt. Hierbei wird ein Abschnitt Δz erst in Umfangsrichtung 46a um einen gesamten Umfang des Implantats 10a beschichtet und danach wird der Beschichtungsstrahl 40a um die Distanz Δz in axialer Richtung 48a weiter in Richtung des zweiten Endes 16a bewegt, um den nächsten Abschnitt Δz' in Umfangsrichtung 46a zu beschichten. Dies wird fortgeführt bis das medizinische Implantat 10a vollständig beschichtet ist.

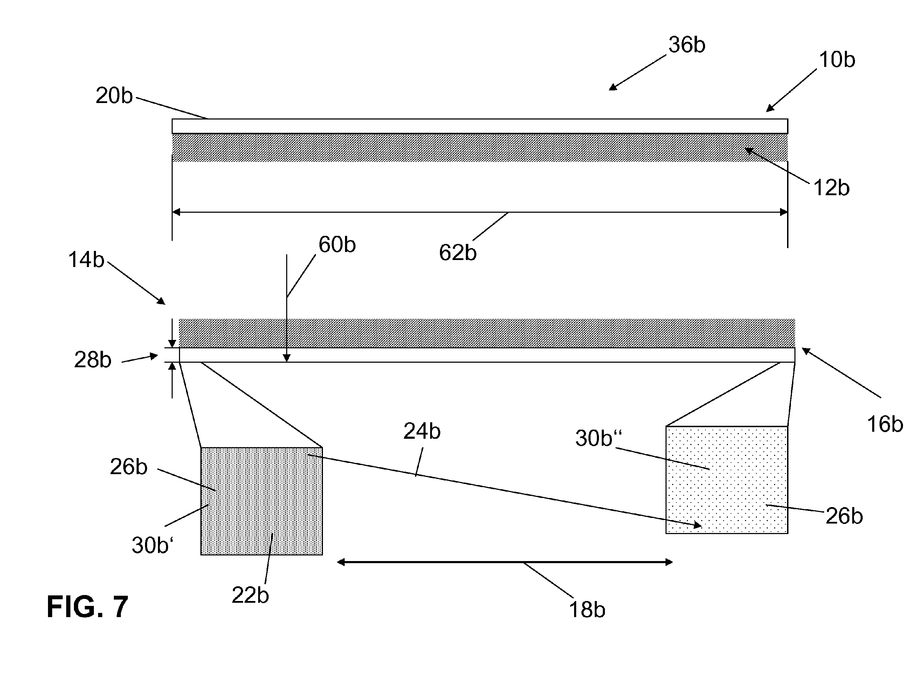

In der Fig. 7 ist ein alternatives Ausführungsbeispiel des medizinischen Implantats 10a dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele sind jedoch den Bezugszeichen der Ausführungsbeispiele die Buchstaben a und b hinzugefügt. Die nachfolgende Beschreibung beschränkt sich im Wesentlichen auf die Unterschiede zu dem Ausführungsbeispiel in den Figuren 1 bis 6, wobei bezüglich gleich bleibender Bauteile, Merkmale und Funktionen auf die Beschreibung des Ausführungsbeispiels in den Figuren 1 bis 6 verwiesen werden kann.

In Fig. 7 ist ein alternatives medizinisches Implantat 10b in der Form eines Stents 36b mit einem Grundkörper 12b aus einem Chrom-Kobalt-Stahl gezeigt. Der Grundkörper 12b hat ein erstes Ende 14b und ein zweites Ende 16b, die in einer Haupterstreckungsrichtung 18b des Grundkörpers 12b an sich gegenüberliegenden Enden des Grundkörpers 12b angeordnet sind. Ferner weist der Grundkörper 12b eine Beschichtung 20b, die als Wirkstoff 22b das ein schmales therapeutisches Fenster aufweisende Immunsuppressivum Sirolimus und eine Polymermatrix 56b aus Poly-L-Lactid auf. Zudem ist der Wirkstoff 22b mit einem Wirkstoffgradienten 24b in die Beschichtung 20b eingebracht, sodass eine Wirkstoffmenge 26b in der Haupterstreckungsrichtung 18b des Grundkörpers 12b von dem ersten Ende 14b zu dem zweiten Ende 16b abnimmt. Diese Abnahme des Wirkstoffgradienten 24b ist mittels mehrerer Wirkstoffkonzentrationen 30b entlang der Beschichtung 20b eingestellt. Am ersten Ende 14b weist die Beschichtung 20b, die über die gesamte Länge 62b in radialer Richtung 60b eine konstante Dicke 28b aufweist, eine Wirkstoffkonzentration 30b' auf, die höhere als die Wirkstoffkonzentration 30b" am zweiten Ende 16b ist.

Die Beschichtung 20b kann mittels eines Verfahrens aufgetragen werden, bei der das Polymer und der Wirkstoff 22b vor der Beschichtung mittels einer geeigneten Mischereinheit (nicht gezeigt) wie beispielsweise einer Gradientenpumpe, einem Mehr-Wege-Ventil, einer Förderschnecke in unterschiedlichen Verhältnissen gemischt wird. Diese Mischereinheit kann auch als Dosiereinheit vorgesehen sein, um die Konzentration des Wirkstoffes zu regulieren.

## Patentansprüche

1. Medizinisches Implantat, mit einem Grundkörper (12a, 12b), der ein erstes Ende (14a, 14b) und ein zweites Ende (16a, 16b) aufweist, die in einer Haupterstreckungsrichtung (18a, 18b) des Grundkörpers (12a, 12b) an sich gegenüberliegenden Enden des Grundkörpers (12a, 12b) angeordnet sind, und einer Beschichtung (20a, 20b), wobei die Beschichtung (20a, 20b) zumindest einen Wirkstoff (22a, 22b) mit einem Wirkstoffgradienten (24a, 24b) aufweist, **dadurch gekennzeichnet, dass** eine Wirkstoffmenge (26a, 26b) zumindest in der Haupterstreckungsrichtung (18a, 18b) des Grundkörpers (12a, 12b) von dem ersten Ende (14a, 14b) zu dem zweiten Ende (16a, 16b) abnimmt.

2. Medizinisches Implantat nach Anspruch1, **dadurch gekennzeichnet, dass** der Wirkstoffgradient (24a) durch eine Dicke (28a', 28a") der Beschichtung (20a) eingestellt ist.

3. Medizinisches Implantat zumindest nach Anspruch 1, **dadurch gekennzeichnet, dass** der Wirkstoffgradient (24b) mittels einer oder mehrerer Wirkstoffkonzentrationen (30b', 30b") entlang der Beschichtung (20b) eingestellt ist.

4. Medizinisches Implantat nach einem der vorhergehenden Ansprüche, **dadurch gekennzeichnet, dass** der zumindest eine Wirkstoff (22a, 22b) ausgewählt ist aus einer Gruppe bestehend aus:
- Lipidregulatoren (Fibrate),
- Immunsuppressiva,
- Immunmodulatoren,
- Vasodilatatoren (Sartane),
- Calciumkanalblocker,
- Calcineurininhibitoren (Tacrolimus),
- Antiphlogistika (Glucocorticoiden, Cortison, Dichlofenac),
- Antünflammatorika (Imidazole),
- Antiallergika,
- Oligonucleoptide (dODN),
- Östrogene (Genistein),
- Endothelbildner (Fibrin),
- Steroide,
- Proteine/Peptide,
- Proliferationshemmer,
- Analgetika,
- Antirheumatika,
- Zytostatika.

5. Medizinisches Implantat nach einem der vorhergehenden Ansprüche, **dadurch gekennzeichnet, dass** der zumindest eine Wirkstoff (22a, 22b) ein schmales therapeutisches Fenster aufweist.

6. Medizinisches Implantat nach einem der vorhergehenden Ansprüche, **dadurch gekennzeichnet, dass** das erste Ende (14a, 14b) in einem implantierten Zustand in einem Hohlraum (32a, 32b) als stromaufwärtiges Ende in einer axialen Fließrichtung (34a, 34b) anordenbar ist.

7. Medizinisches Implantat nach einem der vorhergehenden Ansprüche, **gekennzeichnet durch** eine Ausbildung als Stent (36a, 36b).

8. Medizinisches Implantat nach einem der vorhergehenden Ansprüche, **dadurch gekennzeichnet, dass** der Grundkörper (12a, 12b) zumindest Kobalt und/oder Chrom aufweist.

9. Medizinisches Implantat nach einem der vorhergehenden Ansprüche, **dadurch gekennzeichnet, dass** die Beschichtung (20a, 20b) zumindest ein Polylactid enthält.

10. Medizinisches Implantat nach einem der vorhergehenden Ansprüche, **gekennzeichnet durch** eine Ausgestaltung zur Eluierung zumindest eines Wirkstoffs (22a, 22b) in einem menschlichen und/oder tierischen Körper (38a, 38b), wobei der zumindest eine Wirkstoff (22a, 22b) derart eluierbar ist, dass sich eine gewünschte lokale Konzentrationsverteilung in einer angrenzenden Hohlraumwand (40a, 40b) ergibt.

11. Verfahren, insbesondere Beschichtungsverfahren, zumindest nach Anspruch 1 zur Beschichtung eines medizinischen Implantats (10a) mit einem Grundkörper (12a), der ein erstes Ende (14a) und ein zweites Ende (16a) aufweist, und einer Beschichtung (20a), wobei die Beschichtung (20a) zumindest einen Wirkstoff (22a) mit einem Wirkstoffgradienten (24a) aufweist, **dadurch gekennzeichnet, dass** ein Beschichtungsstrahl (42a) mit einem Polymer/Wirkstoffgemisch (44a) mit einer variablen Geschwindigkeit (*̅v̅*̅) von dem ersten Ende (14a) zu dem zweiten Ende (16a) bewegt wird.

12. Verfahren nach Anspruch 11, **dadurch gekennzeichnet, dass** der Beschichtungsstrahl (42a) mit zunehmender Geschwindigkeit (*̅v̅*̅) von dem ersten Ende (14a) zu dem zweiten Ende (16a) bewegt wird.

13. Verfahren zumindest nach Anspruch 11, **dadurch gekennzeichnet, dass** der Beschichtungsstrahl (42a) relativ zu dem medizinischen Implantat (10a) in Umfangsrichtung (46a) und/oder axialer Richtung (48a) bewegt wird.

14. Verfahren, insbesondere Implantationsverfahren, zur Implantation eines medizinischen Implantats (10a, 10b) in einem tierischen und/oder menschlichen Körper (38a, 38b) zumindest nach Anspruch 1 bis 10 mit einem ersten Ende (14a, 14b) und einem zweiten Ende (16a, 16b), wobei das erste Ende (14a, 14b) eine höhere Wirkstoffbeladung aufweist als das zweite Ende (16a, 16b), **dadurch gekennzeichnet, dass** das erste Ende (14a, 14b) in einem Hohlraum (32a, 32b) als stromaufwärtiges Ende in einer axialen Fließrichtung (34a, 34b) angeordnet wird.
